Europäisches Patentamt

European Patent Office

Office européen des brevets

(19)

(11) **EP 0 927 887 A1**

(12) **EUROPÄISCHE PATENTANMELDUNG**

(43) Veröffentlichungstag:
**07.07.1999 Patentblatt 1999/27**

(51) Int. Cl.⁶: $G01N\ 33/36$, $G01N\ 21/89$, $D01H\ 13/22$

(21) Anmeldenummer: 98122733.3

(22) Anmeldetag: **30.11.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **17.12.1997 CH 289197**

(71) Anmelder: **ZELLWEGER LUWA AG**
**8610 Uster (CH)**

(72) Erfinder: **Felix, Ernst**
**8610 Uster (CH)**

(54) **Verfahren zur Erkennung periodischer Fehler in einem längsbewegten Prüfgut**

(57)    Die Erfindung betrifft ein Verfahren und eine Vorrichtung zur Erkennung periodischer Fehler in einem längsbewegten Prüfgut. Um periodische Fehler in langgestrecktem und längsbewegtem Prüfgut laufend und mit geringem Aufwand erfassen zu können, sollen in zeitlichen Abständen (T1, T2, T3), mindestens zwei in vergleichsweise kurzem Abstand (W/2) aufeinanderfolgende Messungen eines Parameters durchgeführt werden.

Fig. 1

EP 0 927 887 A1

**Beschreibung**

Die Erfindung betrifft ein Verfahren und eine Vorrichtung zur Erkennung periodischer Fehler in einem längsbewegten Prüfgut.

Defekte an Produktionsmaschinen wie z. B. Ringspinnmaschinen verursachen immer wieder periodische Querschnittsschwankungen im Prüfgut, wie z. B. in Garnen. Speziell unrund laufende Vorderzylinder an Ringspinnmaschinen verursachen solche Querschnittsschwankungen. Diese führen zu dem bekannten störenden Moirée-Effekt in den Fertigprodukten. Eine Excentrizität eines Vorderzylinders von z. B. nur 0,1 mm wirkt sich bereits sehr störend aus. Die meisten Vorderzylinder laufen zwar genügend rund. Es gibt aber immer wieder sog. Ausreisser, d.h. Vorderzylinder die grössere Querschnittsschwankungen im Garn verursachen.

Es ist aber sehr schwierig, beispielsweise an Ringspinnmaschinen Produktionsstellen zu erkennen, die Fehler im Garn oder Prüfgut verursachen. Die Excentrizität ist von Auge meistens kaum sichtbar. Im Prinzip wären Messungen mit einer Messuhr möglich. Sie sind aber so umständlich, dass sie sich nur auf Einzelfälle oder die Forschung beschränken.

In der Preis erfolgt die Qualitätskontrolle in der Spinnerei ausschliesslich an den fertigen Garnen. Bis heute wird diese anhand von Stichproben im Labor durchgeführt. Dabei wäre es jedoch äusserst wertvoll die sog. Ausreisser, d.h. die schlecht laufenden Produktionsstellen, zu erfassen. Dies ist aber nur mit einer Vollkontrolle und nicht nur mit Stichproben möglich. Eine Spinnerei weist jedoch zehntausende von Produktionsstellen auf. Die laufende Qualitätskontrolle direkt an jeder Produktionsstelle ist daher wegen der Vielzahl der Produktionsstellen absolut unrealistisch.

Bis heute gibt es weder Verfahren noch Vorrichtungen, die obiges Bedürfnis in irgendeiner Form auch nur einigermassen abzudecken vermögen.

Es ist deshalb eine Aufgabe der Erfindung ein Verfahren und eine Vorrichtung zu schaffen, mit denen periodische Fehler in langgestrecktem und längsbewegtem Prüfgut laufend und mit geringem Aufwand erfasst werden können.

Dies geschieht dadurch, dass in zeitlichen Abständen, mindestens zwei in vergleichsweise kurzem Abstand aufeinanderfolgende Messungen eines Parameters durchgeführt werden. Als Parameter wird vorzugsweise ein Wert aus einer Gruppe von Werten enthaltend den Durchmesser und die Masse des Prüfguts gemessen. Die Vorrichtung enthält mindestens zwei Sensoren, die in einem Abstand zueinander auf einer Bahn vor Produktionseinheiten einer Maschine fahrbar angeordnet sind. Die Sensoren bilden zusammen einen sog. Wandersensor mit mindestens zwei Messorganen, welche vom Prüfgut beim Vorbeifahren des Wandersensors nacheinander an jeder Produktionsstelle jeweils den Querschnitt und/oder Durchmesser erfassen. Die erwähnten Messorgane oder Sensoren weisen einen derartigen Abstand zueinander auf, dass, unter Berücksichtigung der Fahrgeschwindigkeit des Wandersensors und der Produktionsgeschwindigkeit des Prüfguts, derselbe ungefähr der halben Wellenlänge der gesuchten Periode und/oder einem ganzahligen Vielfachen hievon, entspricht. Die jeweils von der gleichen Produktions- oder Spinnstelle erhaltenen Messresultate der Messorgane werden miteinander verknüpft und zugehörig zu jeder Spinnstelle gespeichert, wodurch sich periodische Schwankungen im Garn erkennen lassen. In den Sensoren oder Messorganen erfolgt eine mindestens näherungsweise Erfassung des Garnquerschnittes und/oder Garndurchmessers beim Vorbeifahren des Wandersensors an den Produktionsstellen, wobei der Abstand der Messorgane im Wandersensor so gross ist, dass in der Zeit, in der im Garn eine Länge produziert worden ist, die ungefähr der halben Wellenlänge der entsprechenden Periode und/oder dem ganzzahligen Vielfachen hievon, entspricht, der Wandersensor einen Weg entsprechend dem Abstand der Messorgane zurückgelegt hat. Elektronische Mittel zur Auswertung und Speicherung der Messresultate sind ebenfalls vorgesehen.

Die durch die Erfindung erreichten Vorteile sind insbesondere darin zu sehen, dass damit eine praktisch vollständige Kontrolle aller Produktionsstellen erreicht wird, ohne jeder Produktionsstelle ein eigenes Kontrollelement zuordnen zu müssen.

Im folgenden wird die Erfindung anhand eines Beispiels und mit Bezug auf die beiliegenden Figuren näher erläutert. Es zeigen:

    Figur 1 eine schematische Darstellung der erfindungsgemässen Vorrichtung,

    Figur 2 eine schematische Darstellung des Verfahrens und

    Figur 3 eine weitere Ausführung eines Teils der Vorrichtung.

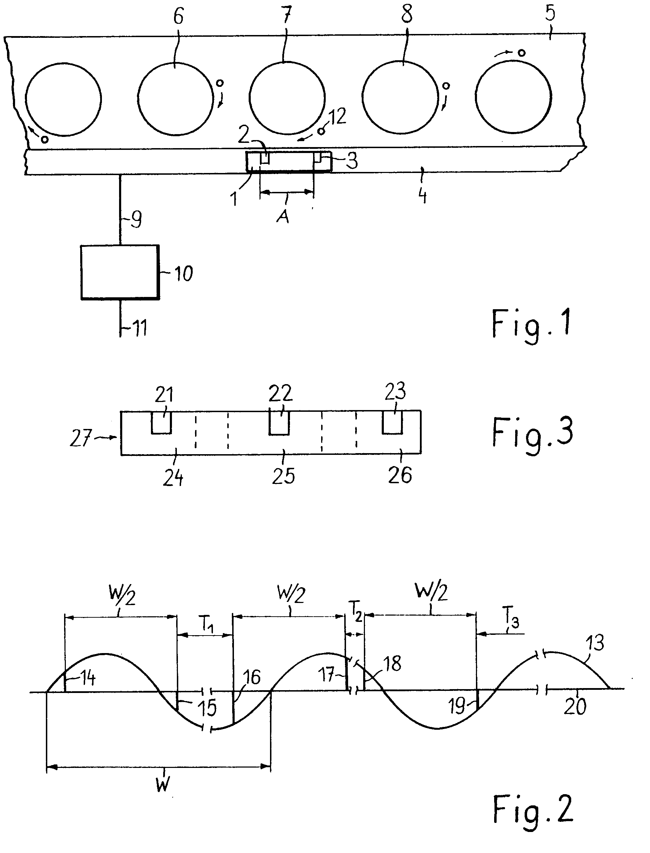

Fig. 1 zeigt schematisch einen Wandersensor 1 mit zwei Messorganen 2 und 3, der auf einer Bahn oder Schiene 4 entlang einer Ringbank 5 mit den Spinnstellen 6, 7 und 8 als Produktionsstellen fahrbar angeordnet ist. Die Messorgane 2, 3 sind über eine Leitung 9 mit einer Auswerteeinheit 10, mit einem Ausgang 11 verbunden. Jedes Messorgan 2, 3 im Wandersensor 1 erfasst das, an der Spinnstelle rotierende Garn 12, beim Vorbeifahren und gibt ein Signal ab, das mindestens näherungsweise proportional dem Garndurchmesser bzw. Garnquerschnitt oder der Masse des Garns bzw. Prüfguts ist.

Bei Ringspinnmaschinen sind in der Regel vor allem periodische Schwankungen von Interesse, die durch fehlerhafte Vorderzylinder in den Streckwerken entstehen. Der Abstand A der Messorgane muss in diesen Fällen so gross sein, dass in der Zeit, in der Garn einer Länge produziert wird, die ungefähr dem halben Umfang der Vorderzylinder entspricht, der Wandersensor 1 einen Weg zurücklegt, der dem Abstand A der Messorgane 2, 3 entspricht.

Wenn z. B. der Umfang der Vorderzylinder 10 cm (Ober- und Unterzylinder sind in der Regel ungefähr gleich gross), d. h. der halbe Umfang 5 cm und die Produktionsgeschwindigkeit 16 cm pro Sekunde beträgt, so ist in 5/16 Sekunden Garn einer Länge produziert worden, die dem halben Umfang des Vorderzylinders entspricht. Beträgt andererseits die Geschwindigkeit des Wandersensors z.B. 20 cm pro Sekunde, so ist der Weg den er in 5/16 Sekunden zurücklegt 6,25 cm lang. Der Abstand A der beiden Messorgane 2, 3 im Wandersensor 1 muss dann 6,25 cm betragen.

Der Abstand muss aber für den vorgesehenen Zweck nicht besonders genau sein. Übliche Schwankungen der Produktionsgeschwindigkeit, die theoretisch jeweils immer einen anderen Abstand erfordern würden, wirken sich nicht störend aus.

Um eine Angabe über periodische Fehler zu erhalten, müssen die Signale oder Werte aus beiden Messorganen in geeigneter Weise ausgewertet oder verknüpft werden.

Zum besseren Verständnis wie eine mathematische Verknüpfung der Signale der beiden Messorgane 2, 3 beispielsweise sein kann, sei folgender hypothetischer Fall angenommen, wie er anhand der Fig. 2 erläutert werden kann:

Ein Garn habe ausschliesslich eine rein periodische Schwankung mit einer Wellenlänge W entsprechend dem Umfang U eines Vorderzylinders. Diese Schwankung ist in Fig. 2 mit 13 bezeichnet und dargestellt. Gemäss der erfindungsgemässen Ausführung nehmen die beiden Messorgane 2, 3 in zeitlichen Abständen T1, T2, T3 usw. mindestens zwei in vergleichsweise kurzem Abstand W/2 aufeinanderfolgende Messungen eines Parameters vor. Der Abstand A im Wandersensor ist so ausgelegt, dass immer im Abstand einer halben Wellenlänge W/2 ein Wertepaar 14, 15, 16, 17 und 18, 19 aufgenommen wird. Allerdings sind die zeitlichen Abstände W/2 in Wirklichkeit wesentlich kleiner als die Abstände T1, T2, T3 usw. die hier lediglich der leichteren Darstellung wegen so kurz aufeinanderfolgen. Es zeigt sich, dass in der Abweichung vom Mittelwert immer ein positiver 14, 17, 18 und ein negativer Wert 15, 16, 19 auftritt. Multipliziert man nun die jeweiligen Messwerte eines Wertepaares, so ergibt sich immer ein negativer Wert. Die mathematische Verknüpfung besteht also darin, dass vorerst mindestens näherungsweise der Mittelwert aller Wertepaare gebildet wird und dann die jeweiligen Abweichungen vom Mittelwert eines Wertepaares miteinander multipliziert werden und diese dann pro Spinnstelle gemittelt werden.

Diese mathematische Verknüpfung ist hergeleitet von der bekannten Theorie der Autokorrelation. In der Literatur ist sie meistens mit Zeitfunktionen dargestellt. Allgemein lautet die Formel für die Autokorrelation:

$$K(\Gamma) = \frac{1}{T} \int_{0}^{T} f(t) \cdot f(t+\Gamma) \cdot dt$$

wobei

K($\Gamma$) = Kortelationsfaktor
f(t) = gegebene Funktion (Zeitfunktion)
$\Gamma$ = Parameter(bei einer Zeitfunktion eine feste Zeit)
T = totale Messzeit

bedeuten.

In unserem Falle liegt keine Zeitfunktion f(t) vor, sondern ein Garnquerschnittsverlauf. Dabei ist die Berechnung der Autokorrelation nicht sinnvoll für den ganzen Garnquerschnitt, sondern nur für die Schwankungen des Garnquerschnittes, d.h. die Abweichungen vom Mittelwert 20. (Als Parameter wird ferner nur ein einziger Wert berechnet, weil in unserem Beispiel nur derjenige von Interesse ist, der dem halben Zylinderumfang und/oder einem ganzahligen Vielfachen hievon entspricht.?)

Im Gegensatz zum obigen hypothetischen Fall weist ein fehlerfreies Garn nur rein zufällige Schwankungen auf. Wird ein solches Garn dem gleichen Prozess unterzogen, so ergibt sich der Wert Null.

Enthält ein Garn sowohl fehlerhafte periodische als auch rein zufällige Schwankungen, so überlagern sich die beiden oben erwähnten Fälle. Je nach der Intensität der periodischen Schwankung wird eine mehr oder weniger negative Abweichung der Werte gegenüber den Werten der Garne ohne diese fehlerhafte periodische Schwankung erhalten. Diese Abweichung zeigt die Intensität der periodischen Schwankung an, wodurch sich die Ausreisser erkennen lassen.

Der Wandersensor 1, der periodisch auf der Schiene 4 in an sich bekannter Weise hin- und herbewegt wird, nimmt nun bei jedem Durchlauf ein Wertepaar von jeder Spinnstelle 6, 7, 8 usw. auf. Diese Werte werden gemittelt und die Abweichungen vom Mittelwert jedes Wertepaares miteinander multipliziert und in bekannter Art elektronisch gespeichert. In einem Tag ergeben sich in der Praxis ca. 1000 Durchläufe des Wandersensors, also 1000 Wertepaare von jeder Spinnstelle.

Die mathematische Verknüpfung kann nun nach folgender Formel vorgenommen werden:

$$K(W/2) = \frac{1}{n} \sum_{i=0}^{i=n} (a_j - \bar{a}) \cdot (b_j - \bar{b})$$

wobei

K(U/2) = Korrelationsfaktor

a$_j$ = jeweiliger Messwert von Sensor a
b$_j$ = jeweiliger Messwert von Sensor b
$\overline{a}$ = Mittelwert aller Werte a
$\overline{b}$ = Mittelwert aller Werte b
n = Anzahl Messwert-Paare

bedeuten.

Der Zeitparameter ($\Gamma$) in der allgemeinen Formel ist hier durch den Abstand der Messorgane gegeben, d. h. entsprechend dem halben Umfang des Vorderzylinders (U/2). Für das obige Beispiel heisst das, dass a$_j$ und b$_j$ um den halben Zylinderumfang zeitverzögerte Werte sind.

Fehlerhafte Garne ergeben Werte, die je nach Intensität des Fehlers in negativer Richtung vom Wert der fehlertosen Garne abweichen. Der Wandersensor erfasst alle oder fast alle Spinnstellen. Diejenigen Spinnstellen, die Werte mit den grössten Abweichungen in die negative Richtung aufweisen, sind die Ausreisser.

Von jeder Spinnstelle werden zwar nur stichprobenweise Wertepaare erhalten. Eventuelle Periodizitäten lassen sich aber mit genügender statistischer Sicherheit erkennen. Damit wird es möglich, in den Spinnereien, die zehn- oder gar hunderttausend Spinnstellen aufweisen, fehlerhafte Spinnstellen zu erkennen und entsprechende Massnahmen zu treffen.

Es muss noch erwähnt werden, dass auch die ganzzahligen Vielfachen des halben Zylinderumfanges die Periodizität anzeigen. Dabei weichen bei ungeradzahligen Vielfachen die Resultate auf die negative, bei geradzahligen Vielfachen auf die positive Seite ab. Allerdings ist dabei eine höhere Genauigkeit des Abstandes der beiden Messorgane notwendig. Zur Erna hung der statistischen Sicherheit des Messresultates können daher auch mehr als zwei Messorgane in einem Wandersensor verwendet werden. Sie müssen lediglich im erwähnten Abstand des ganzzahligen Vielfachen sein. Eine solche Anordnung ist in Fig. 3 gezeigt, bei der drei Sensoren oder Messorgane 21, 22, 23 gezeigt sind, die zudem in Bereichen 24, 25, 26 verstellbar auf dem Wandersensor 27 angeordnet sind, die durch unterbrochene Linien begrenzt sind.

Wenn mehr als nur eine periodische Schwankung untersucht werden soll, können weitere Messorgane eingesetzt werden, wobei deren Abstand zum ersten Messorgan entsprechend den obigen Ausführungen festzulegen ist.

Die ganze Verarbeitung der gemessenen Parameter gemäss den genannten Formeln erfolgt in der Auswerteeinheit 10, die aus einem dementsprechend programmierbaren Prozessor besteht.

Das obige Verfahren mit Vorrichtung ist nicht nur auf Ringspinnmaschinen begrenzt. Es kann überall verwendet werden, wo Wandersensoren eingesetzt werden können.

**Patentansprüche**

1. Verfahren zur Erkennung periodischer Fehler in längsbewegtem Prüfgut (12), dadurch gekennzeichnet, dass in zeitlichen Abständen (T1, T2, T3), mindestens zwei in vergleichsweise kurzem Abstand (W/2) aufeinanderfolgende Messungen einer Kenngrösse des Prüfguts durchgeführt werden.

2. Verfahren nach Anspruch 1, dadurch gekennzeichnet, dass als Kenngrösse der Durchmesser eines Gams als Prüfgut gemessen wird.

3. Verfahren nach Anspruch 1, dadurch gekennzeichnet, dass als Kenngrösse die Masse eines Garns als Prüfgut gemessen wird.

4. Verfahren nach Anspruch 1, dadurch gekennzeichnet, dass aus den Messungen ein Mittelwert gebildet wird.

5. Verfahren nach Anspruch 4, dadurch gekennzeichnet, dass die Messungen in Beziehung zum Mittelwert gesetzt werden und daraus Werte (14 bis 19) für Abweichungen ermittelt werden.

6. Verfahren nach Anspruch 1, dadurch gekennzeichnet, dass aus den Messungen Messwerte gebildet werden, die miteinander verknüpft und anschliessend gespeichert werden.

7. Vorrichtung zur Durchführung des Verfahrens nach Anspruch 1, gekennzeichnet durch, mindestens zwei Sensoren (2, 3), die in einem Abstand (A) zueinander auf einer Bahn (4) vor Produktionseinheiten (6, 7, 8) einer Maschine fahrbar angeordnet sind.

8. Vorrichtung nach Anspruch 7, dadurch gekennzeichnet, dass die Sensoren mit einer Auswerteeinheit (10) verbunden sind.

9. Vorrichtung nach Anspruch 7, dadurch gekennzeichnet, dass die Sensoren (21, 22, 23) verschiebbar angeordnet sind.

10. Verfahren nach Anspruch 1, dadurch gekennzeichnet, dass die Kenngrössen an einer Vielzahl gleichartiger Prüfgüter an nebeneinanderliegenden Produktionsstellen (6, 7, 8) nacheinander erfasst werden.

Fig.1

Fig.3

Fig.2

Europäisches
Patentamt

# EUROPÄISCHER RECHERCHENBERICHT

Nummer der Anmeldung

EP 98 12 2733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | FR 2 338 884 A (RIETER AG MASCHF)<br>19. August 1977<br>* Seite 10, Zeile 50 - Seite 14, Zeile 32;<br>Ansprüche 1-4; Abbildungen 4,6,7 * | 1-8 | G01N33/36<br>G01N21/89<br>D01H13/22 |
| A | DE 27 50 152 A (ZELLWEGER USTER AG)<br>28. September 1978<br>* Seite 7, Absatz 2 - Seite 8, Absatz 1 * | 1 | |
| A | EP 0 594 220 A (TOKYO SHIBAURA ELECTRIC CO) 27. April 1994<br>* Seite 2 - Seite 3 *<br>* Seite 5, Zeile 11 - Seite 6, Zeile 10;<br>Ansprüche 1,2; Abbildungen 5-7 * | 1 | |
| A | EP 0 358 236 A (FUJI PHOTO FILM CO LTD)<br>14. März 1990<br>* Seite 2 - Seite 3; Ansprüche 1-3 * | 1 | |
| A | WO 89 00215 A (ZELLWEGER USTER AG)<br>12. Januar 1989<br>* Ansprüche 1,6 * | 1 | RECHERCHIERTE SACHGEBIETE (Int.Cl.6) |
| A | DE 43 35 262 A (MURATA MACHINERY LTD)<br>21. April 1994<br>* Spalte 1 - Spalte 3 * | 1 | G01N<br>D01H |

Der vorliegende Recherchenbericht wurde für alle Patentansprüche erstellt

| Recherchenort | Abschlußdatum der Recherche | Prüfer |
|---|---|---|
| DEN HAAG | 12. März 1999 | Tabellion, M |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**     EP 98 12 2733

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-03-1999

| Im Recherchenbericht angeführtes Patentdokument | Datum der Veröffentlichung | Mitglied(er) der Patentfamilie | Datum der Veröffentlichung |
|---|---|---|---|
| FR 2338884 A | 19-08-1977 | CH 612152 A | 13-07-1979 |
| | | DE 2649779 A | 28-07-1977 |
| | | GB 1557512 A | 12-12-1979 |
| | | JP 1307329 C | 13-03-1986 |
| | | JP 52091936 A | 02-08-1977 |
| | | JP 60028933 B | 08-07-1985 |
| | | NL 7700256 A | 28-07-1977 |
| | | US 4058962 A | 22-11-1977 |
| DE 2750152 A | 28-09-1978 | CH 615404 A | 31-01-1980 |
| | | BE 863392 A | 16-05-1978 |
| | | CS 197308 B | 30-04-1980 |
| | | GB 1597553 A | 09-09-1981 |
| | | IN 149808 A | 24-04-1982 |
| | | JP 53117461 A | 13-10-1978 |
| | | JP 59042801 B | 17-10-1984 |
| | | US 4168604 A | 25-09-1979 |
| EP 0594220 A | 27-04-1994 | JP 1136054 A | 29-05-1989 |
| | | JP 2054836 C | 23-05-1996 |
| | | JP 7086478 B | 20-09-1995 |
| | | JP 1136055 A | 29-05-1989 |
| | | JP 2054837 C | 23-05-1996 |
| | | JP 7086479 B | 20-09-1995 |
| | | DE 3851609 D | 27-10-1994 |
| | | DE 3851609 T | 19-01-1995 |
| | | DE 3855913 D | 19-06-1997 |
| | | DE 3855913 T | 04-12-1997 |
| | | EP 0316961 A | 24-05-1989 |
| | | US 4958307 A | 18-09-1990 |
| EP 0358236 A | 14-03-1990 | JP 2051326 C | 10-05-1996 |
| | | JP 2074852 A | 14-03-1990 |
| | | JP 7086474 B | 20-09-1995 |
| | | DE 68928510 D | 05-02-1998 |
| | | DE 68928510 T | 16-04-1998 |
| | | US 4982600 A | 08-01-1991 |
| WO 8900215 A | 12-01-1989 | CH 675133 A | 31-08-1990 |
| | | EP 0322430 A | 05-07-1989 |
| | | IN 173071 A | 05-02-1994 |
| | | JP 2500117 T | 18-01-1990 |
| | | US 5030841 A | 09-07-1991 |
| DE 4335262 A | 21-04-1994 | JP 2611611 B | 21-05-1997 |
| | | JP 6128819 A | 10-05-1994 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**                    EP 98 12 2733

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-03-1999

| Im Recherchenbericht angeführtes Patentdokument | Datum der Veröffentlichung | Mitglied(er) der Patentfamilie | Datum der Veröffentlichung |
|---|---|---|---|
| DE 4335262 A | | IT 1266500 B | 30-12-1996 |
| | | US 5592849 A | 14-01-1997 |